# EUROPEAN PATENT APPLICATION

(11) **EP 0 938 888 A2**
(43) Date of publication of application: **01.09.1999**
(21) Application number: 99102414.2
(22) Date of filing: 08.02.1999
(51) Int. Cl.: A61K 7/00, A61K 7/13, A61K 7/135

(54) **Two-formulation mixed cosmetic**

(30) Priority: 09.02.1998 JP 4291398
(71) Applicant: SHISEIDO COMPANY LIMITED, Chuo-ku, Tokyo 104-8010 (JP)
(72) Inventor: Suzuki, Kazunobu, Shiseido Research Center 1, Yokohama-shi, Kanagawa 223-8553 (JP); Yasuda, Masaaki, Shiseido Research Center 1, Yokohama-shi, Kanagawa 223-8553 (JP)
(74) Representative: TER MEER STEINMEISTER & PARTNER GbR

(57) **Abstract**

Two mixed cosmetic comprising formulation (I) which has a pH of 7 or higher and contains a carboxyvinyl polymer or modified carboxyvinyl polymer, lower alcohol, and higher fatty acid and formulation (II), the mixture of said formulation (I) and formulation (II) having a pH of 5 or higher, wherein the cosmetic main agent is contained in one or both of said formulation (I) and said formulation (II).

As clearly shown in the above description, the 2-formulation mixed cosmetic of the present invention can provide a cosmetic which has a low viscosity and is easy to handle before use, yet at the time of use develops sufficient viscosity to stay on the applied site, does not limit the flexibility of the recipe design, and also is stable.

## Description

### Related Application

This application claims the priority of Japanese Patent application No.10-42913 filed on February 9, 1998, which is incorporated herein by reference.

### Background of the Invention

### 1. Field of the invention

This invention relates in general to a 2-formulation mixed cosmetic which is used by mixing formulation (I) and formulation (II) immediately preceding use, and more particularly to a 2-formulation mixed cosmetic which exhibits an increase in viscosity by mixing formulation (I) and formulation (II).

### 2. The Prior Art

In the field of cosmetics such as hair dyes, a 2-formulation mixed cosmetic has been used which is composed of a formulation (I) and formulation (II) preserved in separate containers and mixed immediately preceding use. In such a 2-formulation mixed cosmetic, the effective ingredient of the cosmetic is formed by the mixing of formulation (I) and formulation (II). This is often used when the effective ingredient is unstable and needs to be used immediately after it is formed. Examples of such an ingredient include oxidation hair dyes, hair bleaches, and enzyme-containing agents.

For these 2-formulation mixed cosmetics, the viscosity needs to be low before mixing in order to reduce the amount of the cosmetic remaining in the container due to adhesion to the inner walls of the container and also to facilitate the mixing procedure of formulation (I) and formulation (II). On the other hand, when applying the mixed formulation (I) and formulation (II), the mixture needs to have a prescribed viscosity in order for the mixture to stay on the applied site for a certain amount of time. Conventionally, in order to prepare a cosmetic with a prescribed viscosity, one or both of formulation (I) and formulation (II) have been prepared as an emulsifying agent or a viscous liquid agent so that a prescribed viscosity can also be obtained after mixing. A prescribed viscosity has also been achieved by adding a surfactant to one or both of formulation (I) and formulation (II) and utilizing the viscosity which develops from the concentration change due to the mixing of surfactant solutions. Also, for enzyme-containing toilet water, viscosity development due to dissolution and dispersal of a powder substance has been utilized.

Furthermore, Japanese unexamined patent publications Tokkai Hei 7-82122 and Tokkai Hei 7-82123 disclose a 2-formulation mixed cosmetic which uses a non-water soluble anionic polymer for the thickener. In these, the non-water soluble anionic polymer is added to a water base dispersing agent and a cationic polymer is added to an alkaline water base lotion. At the time of use, the mixing of the water base dispersing agent and the alkaline water base lotion results in a reaction between the anionic polymer and cationic polymer, forming a gel.

However, when preparing one or both of formulation (I) and formulation (II) as an emulsifying agent or a viscous liquid agent there is a problem in that, since a high viscosity develops even before the mixing, the drug stick to the inner walls of the container, leading to a large amount of the cosmetic remaining in the container and making it difficult to mix formulation (I) and formulation (II). Also, in the case where viscosity development due to the concentration change in the surfactant solution is utilized, not only is the achieved viscosity not enough but the viscosity is significantly influenced by the ingredients in the recipe. Because of this, there is a problem in that selection of the ingredients is significantly limited, making it difficult to design a recipe which satisfactorily exhibits the essential functions of the cosmetic. Furthermore, in the case where viscosity development due to dissolution and dispersion of a powder substance is utilized for an enzyme-containing toilet water, there is a problem in that very little increase in the viscosity is achieved.

Also, in the case of the oxidation hair dye compound described in Tokkai Hei 7-82122 and Tokkai Hei 7-82123, a gel is formed through the reaction between the thickeners, i.e. the anionic polymer and the cationic polymer. Because of this, if another anionic ingredient is added to the alkaline water base lotion then it will react with this cationic polymer, making it impossible to thicken the anionic polymer, which is the thickener. Therefore, the selection of the ingredients in the alkaline water base lotion is significantly restricted, limiting the flexibility in pharmaceutical preparations. Also, this anionic polymer, which is the thickener, is not soluble in water. Therefore, in order to be prepared as a cosmetic, it has to be in the form of a dispersion system such as a suspension and a stable formulation is difficult to obtain in this way. Also, since use of an anionic polymer as a thickener results in formation of a gel, a specific desired viscosity according to a purpose cannot be achieved, which narrows the range of use.

The present invention is carried out based on the problems described above and its object is to provide a 2-formulation mixed cosmetic which has a low viscosity and is easy to handle before use, yet at the time of use develops a sufficient viscosity to stay on the applied site, does not limit the flexibility of the recipe design, and also can provide a stable cosmetic.

### Brief Summary of the Invention

The inventors conducted earnest research to achieve the object described above and discovered that the aforementioned problems can be solved by adding appropriate amounts of a carboxyvinyl polymer or modified carboxyvinyl polymer, lower alcohol, and higher fatty acid to formulation (I), thus completing the present invention.

That is, the present invention is a 2-formulation mixed cosmetic comprising formulation (I) which has a pH of 7 or higher and contains a carboxyvinyl polymer or modified carboxyvinyl polymer, lower alcohol, and higher fatty acid and formulation (II), the mixture of said formulation (I) and formulation (II) having a pH of 5 or higher, wherein the cosmetic main agent is contained in one or both of said formulation (I) and said formulation (II).

### Detailed Description of the Invention

Formulation (I) of the present invention has a pH of 7 or higher, and contains a carboxyvinyl polymer or modified carboxyvinyl polymer, lower alcohol, and higher fatty acid. Usually, a carboxyvinyl polymer exhibits a sudden increase in viscosity at around neutral pH or on the alkaline side. However, the addition of an appropriate amount of a lower alcohol and higher fatty acid decreases its viscosity so that the viscosity would increase after the mixing with formulation (II). The selection of formulation (II) of the present invention is not limited as long as it preferably has a pH of 5 or lower and the pH of the mixture after mixing with formulation (I) is 5 or higher.

The type of carboxyvinyl polymer used in the present invention is not limited provided that it has the aforementioned characteristics. Examples of the modified carboxyvinyl polymer include alkylated carboxyvinyl polymer. Examples of the carboxyvinyl polymer and modified carboxyvinyl polymer with such characteristics include a product named "Carbopol" and "Pemulen" from Goodrich Company and a product named "Hiviswako" from Wako Pure Chemical Industries, Ltd. The blend ratio of the carboxyvinyl polymer in formulation (I) can be adjusted according to the type of pharmaceutical preparation and the required viscosity. However, in general, if the blend ratio of the carboxyvinyl polymer is less than 0.005 wt% of the total formulation (I) then a sufficient viscosity cannot be obtained, whereas if it is more than 5.0 wt% then the viscosity will be too high. Therefore, generally, 0.005-5 wt% is preferable and 0.1-3.0 wt% is more preferable.

For the lower alcohol in the present invention, one or more selected from a group consisting of methanol, ethanol, butanol, propanol, isopropanol, isobutyl alcohol, and t-butyl alcohol can be used. The blend ratio of the lower alcohol in formulation (I) can be adjusted according to the type of pharmaceutical preparation and the required viscosity. A preferable blend ratio is 0.01-25.0 wt%, more preferably 5.0-20.0 wt%, of the total formulation (I). If the blend ratio of the lower alcohol is less than 0.01 wt% then the viscosity will be too high. If it is more than 25.0 wt% then a sufficient viscosity cannot be obtained after the mixing.

The higher fatty acid for use in the present invention is preferably one of those with a carbon number of 12-24. Examples include lauric acid, myristic acid, palmitic acid, stearic acid, behenic acid, oleic acid, isostearic acid, linolic acid, and linolenic acid. The blend ratio of the higher fatty acid in formulation (I) can be adjusted according to the type of pharmaceutical preparation and the required viscosity. A preferable blend ratio is 0.01-25.0 wt%, more preferably 5.0-15.0 wt%, of the total formulation (I). If the blend ratio of the higher fatty acid is less than 0.01 wt% then the viscosity will be too high. If it is more than 25.0 wt% then a sufficient viscosity cannot be obtained after the mixing.

The pH adjustment of formulation (I) and formulation (II) can be done by using a pH adjustment agent commonly used in the cosmetic field. Examples for use as the acid include inorganic acids (such as phosphoric acid and hydrochloric acid) and organic acids (such as citric acid, tartaric acid, and malic acid). Examples for use as the alkaline agent include ammonia, alkanol amines (such as ammonia, monoethanolamine, diethanolamine, and tritethanolamine), basic amino acids (such as arginine and lysine), organic amines (such as guanidine and 2-amino-2-methylpropane), as well as inorganic alkalis (such as sodium hydroxide and potassium hydroxide). In addition, a buffer solution which is a combination of these with salts of them (such as phosphoric acid-sodium salt of phosphoric acid) is used.

The mixing ratio of formulation (I) and formulation (II) is not limited in particular as long as the efficacy and/or stability of the cosmetic main agent and the carboxyvinyl polymer are not affected. Usually, it is 2 : 1-1 : 3 (weight ratio of formulation (I) : formulation (II)), preferably 1 : 1-1 : 2. The 2-formulation mixed cosmetic pertaining to the present invention can be used for various purposes such as hair dyes, hair bleaches, permanent wave agents, depilatories, and enzyme-containing toilet waters.

Examples of the cosmetic main ingredient include dyes for use as a hair dye such as oxidation dye, direct dye, acidic dye, and basic dye, oxidation agents for use as a hair bleach, and reducing agent for use as a permanent wave agent and depilatory.

Of these, examples of the oxidation dyes include p-diamines which have one or more types of NH₂- group, NHR¹- group or N(R¹)₂- group (R¹ is an alkyl or hydroxyalkyl group with a carbon number of 1-4) [such as p-phenylenediamine, p-toluilenediamine, N-methyl-p-phenylenediamine, N,N-dimethyl-p-phenylenediamine, N,N-diethyl-2methyl-p-phenylenediamine, N-ethyl-N-(hydroxyethyl)-p-phenylenediamine, chloro-p-phenylenediamine, N,N-bis-(2-hydroxyethyl)-p-phenylenediamine, methoxy-p-phenylenediamine, 2,6-dichloro-p-phenylenediamine, 2-chloro-6-bromo-p-phenylenediamine, 2-chloro-6-methyl-p-phenylenediamine, 6-methoxy-3-methyl-p-phenylenediamine, 2,5-diaminoanisole, N-(2-hydroxypropyl)-p-phenylenediamine and N-2-methoxy-p-phenylenediamine]; diaminopyridine derivatives [such as 2,5-diaminopyridine]; aminophenols [such as paraaminophenol, 2-methyl-4-aminophenol, 3-methyl-4-aminophenol, 2-chloro-4-aminophenol, 3-chloro-4-aminophenol, 2,6-dimethyl-4-aminophenol, 3,5-dimethyl-4-aminophenol, 2,3-dimethyl-4-aminophenol, 2,5-dimethyl-4-aminophenol, 2,4-diaminophenol, 5-aminosalicylic acid, o-aminophenol]; hydroxynaphthalenes [such as α -naphtol, 1,5-dihydroxynaphthalene, 1,7-dihydroxynaphthalene]; hydroxybenzenes [such as o-cresol, m-cresol, 2,6-dimethylphenol, 2,5-dimethylphenol, 3,4-dimethylphenol, 3,5-dimethylphenol, pyrogallol, 5-amino-2-methylphenol, hydroquinone, 4-aminophenol, m-aminophenol]; m-phenylenediamines [such as 2,4-diaminoanisole, m-toluilenediamine, m-phenylenediamine]; resorcinol derivatives [such as resorcinol, resorcinolmonomethylether, 4-chlororesorcinol, 2-methylresorcinol]; pyrazolone derivatives [such as 1-phenyl-3-methyl-5-pyrazolone, 1-phenyl-3-amino-5-pyrazolone]; fluoro-substituted diaminos [such as 3,5-diaminotrifluoromethylbenzene, 2,4-diamino-fluorobenzene, 3,5-diamino-fluorobenzene]; pyrimidine derivatives [such as 2,4-diamino-6-dihydroxypyrimidine, 2,4,6-triaminopyrimidine, 2-amino-4,6-dihydroxypyrimidine, 4-amino-2,6-dihydroxypyrimidine, 4,6-diamino-2-hydroxypyrimidine, 2,6-diaminopyrimidine]; others [such as benzcatechin, 1-phenyl-3,5-dimethylpyrazolidine, 1-methyl-7-dimethyl-amino-4-hydroxy-2-chinolon, 2,4-diaminophenoxyethanol, o-phenylenediamine].

Examples of the direct dyes include 2-amino-4-nitrophenol, 2-amino-5-nitrophenol, nitro-p-phenylenediamine hydrochloride, nitro-p-phenylenediamine, p-aminophenylsulfamic acid, p-nitro-o-phenylenediamine, picramic acid, sodium picramate, picric acid, chrome brown RH, hematin, nitro-p-phenylenediamine sulfate salt, p-nitro-o-phenylenediamine sulfate, p-nitro-m-phenylenediamine sulfate, 1-amino-4-methylaminoanthraquinone and 1,4-diaminoanthraquinone.

Examples of the acidic dyes include red 2, red 3, red 102, red 104, red 105, red 106, yellow 4, yellow 5, green 3, blue 1, blue 2, red 201, red 227, red 230, red 231, red 232, orange 205, orange 207, yellow 202, yellow 203, green 201, green 204, green 205, blue 202, blue 203, blue 205, brown 201, red 401, red 502, red 503, red 504, red 506, orange 402, yellow 402, yellow 403, yellow 406, yellow 407, green 401, green 402, violet 401 and black 401.

Examples of oil soluble dyes including red 215, red 218, red 225, orange 201, orange 206, yellow 201, yellow 204, green 202, violet 201, red 501, red 505, orange 403, yellow 404, yellow 405 and blue 403.

Examples of the basic dyes such as red 213 and red 214; and basic dyes from Arianor, Inc. such as Sienna Brown, Mahogany, Madder Red, Steel Blue and Straw Yellow.

Examples of the oxidizing agents for use as a hair bleach include hydrogen peroxide and bromate.

Examples of the main agent for the permanent wave agent and the depilatory are reducing agents such as thioglycolic acid, thioglycolate, cysteine, glyceryl thioglycolate, thioglycerine and cysteamine thiosulfate.

These cosmetic main agents can be blended in either or both of formulation (I) and formulation (II).

In the present invention, other ingredients used in common cosmetics can be used in either or both of formulation (I) and formulation (II) within the range where the effect of the present invention is not affected. Examples of these include perfumes, alcohols such as aromatic alcohols and polyhydric alcohols, cationic surfactants, anionic surfactants, nonionic surfactants, ampholytic surfactants, preservatives, ultraviolet light absorbents, sequestering agents, and reducing agents.

As clearly shown in the above description, the 2-formulation mixed cosmetic of the present invention can provide a cosmetic which has a low viscosity and is easy to handle before use, yet at the time of use develops sufficient viscosity to stay on the applied site, does not limit the flexibility of the recipe design, and also is stable.

### Examples

Examples of the present invention are described below. The present invention is not limited to these examples. The amount of each ingredient is indicated in weight percent units in formulation (I) or formulation (II) unless specified otherwise.

### Examples 1-3 and Comparative examples 1-3

Based on the recipes shown in Table 1, a hair dye consisting of formulation (I) and formulation (II) was prepared with the method described later. The pH and viscosity of formulation (I) and the pH of formulation (II) were measured. The obtained formulation (I) and formulation (II) were mixed at a ratio of 1 : 1.5 (weight ratio of formulation (I) : formulation (II)) and the mixture was evaluated for its viscosity, ease of mixing, drip resistance, and the dyeing potency and usability (smoothness) when applied on the hair, using the criteria shown below. The results are shown in Table 2.
(1) Method of evaluating the viscosity after mixing
   ⓞ : The mixture has an adequate viscosity, is easy to get out from the application container and easy to handle.
   ○ : The mixture has a rather high viscosity and hard to get out from the application container.
   Δ : The mixture has a rather low viscosity and drips from the spout of the application container.
   × : The mixture has a very low viscosity and drips from the spout of the application container, making it impossible to apply it on the hair.
(2) Method of evaluating ease of mixing
   ⓞ : Well mixed after combining them and shaking the mixture slowly up and down 15 times.
   ○ : Well mixed after combining them and shaking the mixture slowly up and down 30 times.
   Δ : Well mixed after combining them and shaking the mixture vigorously up and down 30 times.
   × : Not well mixed even after shaking the mixture up and down vigorously.
(3) Method of evaluating drip resistance
   ⓞ : No dripping is observed 30 minutes after the application.
   ○ : Some dripping is observed 30 minutes after the application.
   Δ : Some dripping is observed 15 minutes after the application.
   × : Dripping is observed immediately after the application.
(4) Method of evaluating dyeing potency
   ⓞ : Dyeing potency is very high.
   ○ : Dyeing potency is high.
   Δ : Dyeing potency is low.
   ×: Almost no dyeing occurs.
(5) Method of evaluating usability
   ⓞ : Very smooth and combing is easy.
   ○ : Smooth and combing is easy.
   Δ : Squeaky and combing is not easy.
   × : Squeaky and combing is impossible.

### (1) Preparation method

### (formulation (I))

After dissolving the carboxyvinyl polymer in ion exchanged water, the ingredients were added one after another and stirred/dissolved.

### (formulation (II))

The ingredients were added one after another into ion exchanged water and stirred/dissolved.

**[Table 1]**

| Ingredient | Example | | | Comparative example | | |
|---|---|---|---|---|---|---|
| | 1 | 2 | 3 | 1 | 2 | 3 |
| A. Formulation (I) | | | | | | |
| Carboxyvinyl polymer | 1.0 | 2.0 | 3.0 | - | 2.0 | 1.5 |
| Stearic acid | 5.0 | 3.0 | 10.0 | 15.0 | - | 5.0 |
| Ethanol | 10.0 | 8.0 | 15.0 | 5.0 | 10.0 | - |
| Aqueous ammonia | Appropriate amount | Appropriate amount | Appropriate amount | Appropriate amount | Appropriate amount | Appropriate amount |
| Antioxidant | Appropriate amount | Appropriate amount | Appropriate amount | Appropriate amount | Appropriate amount | Appropriate amount |
| Chelating agent | Appropriate amount | Appropriate amount | Appropriate amount | Appropriate amount | Appropriate amount | Appropriate amount |
| Paraphenylenediamine | 0.5 | 1.0 | 0.8 | 1.0 | 0.5 | 0.8 |
| Resorcinol | 0.8 | 0.2 | 0.5 | 0.4 | 0.5 | 0.3 |
| Paraaminoorthocresol | 0.2 | 0.5 | 0.1 | 0.3 | 0.2 | 0.5 |
| Metaaminophenol | 0.1 | 0.2 | 0.3 | 0.1 | 0.1 | 0.4 |
| Ion exchanged water | Balance | Balance | Balance | Balance | Balance | Balance |
| pH | 10.0 | 9.5 | 8.5 | 10.0 | 9.0 | 9.5 |
| Viscosity (cps) | 500 | 900 | 700 | 300 | > 100.000 | > 100,000 |

| B. Formulation (II) | | | | | | |
|---|---|---|---|---|---|---|
| Aqueous hydrogen peroxide 30% | 20.0 | 20.0 | 20.0 | 20.0 | 20.0 | 20.0 |
| Citric acid | Appropriate amount | Appropriate amount | Appropriate amount | Appropriate amount | Appropriate amount | Appropriate amount |
| Chelating agent | Appropriate amount | Appropriate amount | Appropriate amount | Appropriate amount | Appropriate amount | Appropriate amount |
| Ion exchanged water | Balance | Balance | Balance | Balance | Balance | Balance |
| pH | 3.0 | 2.8 | 2.9 | 3.0 | 3.5 | 2.8 |

**[Table 2]**

| Ingredient | Example | | | Comparative example | | |
|---|---|---|---|---|---|---|
| | 1 | 2 | 3 | 1 | 2 | 3 |
| Viscosity after mixing | ⓞ | ⓞ | ⓞ | × | ○ | ○ |
| Ease of mixing | ⓞ | ⓞ | ⓞ | ○ | × | × |
| Drip resistance | ⓞ | ⓞ | ⓞ | × | Δ | Δ |
| Dyeing potency | ⓞ | ⓞ | ⓞ | Δ | Δ | Δ |
| Usability | ⓞ | ⓞ | ⓞ | × | × | × |

### Example 4 Hair bleach

| Formulation (I) | |
|---|---|
| Carboxyvinyl polymer | 1.5 wt% |
| Oleic acid | 10.0 |
| Propanol | 10.0 |
| Aqueous ammonia | Appropriate amount |
| Triethanolamine | Appropriate amount |
| Chelating agent | Appropriate amount |
| Perfume | 0.1 |
| Hydrolyzed protein | 0.1 |
| Silicone | 0.1 |
| Ion exchanged water | Balance |

| Formulation (II) | |
|---|---|
| Aqueous hydrogen peroxide 30% | 20.0 |
| Tartaric acid | Appropriate amount |
| Chelating agent | Appropriate amount |
| Ion exchanged water | Balance |

### (Preparation method)

After dissolving the carboxyvinyl polymer in ion exchanged water, the ingredients were added one after another and stirred/dissolved (formulation (I), viscosity: 400 cps). The ingredients were added one after another into ion exchanged water and stirred/dissolved (formulation (II)).

The obtained formulation (I) and formulation (II) were easy to mix and the mixture was not easy to drip and had a strong bleaching capability.

### Example 5 Hair bleach

| Formulation (I) | |
|---|---|
| Carboxyvinyl polymer | 1.0 wt% |
| Linolic acid | 15.0 |
| Butanol | 3.0 |
| Methanol | 3.0 |
| Sodium hydroxide | Appropriate amount |
| Chelating agent | Appropriate amount |
| Perfume | 0.1 |
| Ion exchanged water | Balance |

| Formulation (II) | |
|---|---|
| Aqueous hydrogen peroxide 30% | 20.0 |
| Hydrochloric acid | Appropriate amount |
| Chelating agent | Appropriate amount |
| Ion exchanged water | Balance |

### (Preparation method)

After dissolving the carboxyvinyl polymer in ion exchanged water, the ingredients were added one after another and stirred/dissolved (formulation (I), viscosity: 350 cps). The ingredients were added one after another into ion exchanged water and stirred/dissolved (formulation (II)).

The obtained formulation (I) and formulation (II) were easy to mix and the mixture was drip resistant and had a strong bleaching capability.

### Example 6 Hair bleach

| Formulation (I) | |
|---|---|
| Alkylated carboxyvinyl polymer | 1.0 wt% |
| Linolic acid | 15.0 |
| Butanol | 3.0 |
| Methanol | 3.0 |
| Sodium hydroxide | Appropriate amount |
| Chelating agent | Appropriate amount |
| Perfume | 0.1 |
| Ion exchanged water | Balance |

| Formulation (II) | |
|---|---|
| Aqueous hydrogen peroxide 30% | 20.0 |
| Hydrochloric acid | Appropriate amount |
| Chelating agent | Appropriate amount |
| Ion exchanged water | Balance |

### (Preparation method)

After dissolving the alkylated carboxyvinyl polymer in ion exchanged water, the ingredients were added one after another and stirred/dissolved (formulation (I), viscosity: 350 cps). The ingredients were added one after another into ion exchanged water and stirred/dissolved (formulation (II)).

The obtained formulation (I) and formulation (II) were easy to mix and the mixture was drip resistant and had a strong bleaching capability.

## Claims

1. Two-formulation mixed cosmetic comprising formulation (I) which has a pH of 7 or higher and contains a carboxyvinyl polymer or modified carboxyvinyl polymer, lower alcohol, and higher fatty acid and formulation (II), the mixture of said formulation (I) and formulation (II) having a pH of 5 or higher, wherein the cosmetic main agent is contained in one or both of said formulation (I) and said formulation (II).

2. The 2-formulation mixed cosmetic of claim 1 wherein said formulation (II) has a pH of 5 or lower.

3. The 2-formulation mixed cosmetic of claim 1 or 2 wherein the viscosity of said formulation (I) is 3,000 cps or less.

4. The 2-formulation mixed cosmetic of any of claims 1 through 3 wherein said cosmetic main agent is a dye.

5. The 2-formulation mixed cosmetic of claim 4 wherein said cosmetic main agent is an oxidation dye contained in said formulation (I) and said formulation (II) contains an oxidizing agent.
